Europäisches Patentamt

European Patent Office

Office européen des brevets

(19)

(11) Veröffentlichungsnummer: **0 431 543 A1**

(12) # EUROPÄISCHE PATENTANMELDUNG

(21) Anmeldenummer: 90123154.8

(22) Anmeldetag: 04.12.90

(51) Int. Cl.5: **C12N 15/54**, C12N 15/81,
C12N 1/18, C12N 1/21,
//(C12N1/21,C12R1:865)

(30) Priorität: 08.12.89 DE 3940651

(43) Veröffentlichungstag der Anmeldung:
12.06.91 Patentblatt 91/24

(84) Benannte Vertragsstaaten:
AT BE CH DE DK ES FR GB GR IT LI LU NL SE

(71) Anmelder: BEHRINGWERKE
Aktiengesellschaft
Postfach 1140
W-3550 Marburg 1(DE)

(72) Erfinder: Bröker, Michael, Dr.
Lindenweg 16
W-3550 Marburg(DE)
Erfinder: Grote, Mathias, Dr.
Gladenbacher Weg 65
W-3550 Marburg(DE)

(74) Vertreter: Klein, Otto, Dr. et al
Hoechst AG Zentrale Patentabteilung
Postfach 80 03 20
W-6230 Frankfurt am Main 80(DE)

(54) **Verfahren zur Expression von Fremdgenen in Hefen.**

(57) Die Erfindung betrifft die Optimierung der Expression von Fremdgenen, insbesondere rekombinantem Faktor xIIIa (rFXIIIa) in Hefen. Die Optimierung erfolgte im wesentlichen durch Einsatz eines hybriden Gal $_{UAS}$/Cycl Promotors, Deletion der 5'-nichttranslatierten Region sowie Verkürzung der 3'-nichttranslatierten Region der FXIIIa cDNA, Auswahl von speziellen Hefestämmen und ein besonders geeignetes Wachstumsmedium aus Molke bzw. Molkehydrolysat. In Schüttelkulturen sind so Mengen von ≧ 100 mg/l FXIIIa erzielbar, in Fermentation nach "scale-up" ≧ 500 mg/l.

EP 0 431 543 A1

# VERFAHREN ZUR EXPRESSION VON FREMDGENEN IN HEFEN

Die Erfindung betrifft die Optimierung der Expression von Fremdgenen, insbesondere rekombinantem Faktor XIIIa (rFXIIIa) in Hefen. Die Optimierung erfolgte im wesentlichen durch Einsatz eines hybriden Gal $_{UAS}$/Cycl Promotors, Deletion der 5'-nichttranslatierten Region sowie Verkürzung der 3'-nichttranslatierten Region der FXIIIa cDNA, Auswahl von speziellen Hefestämmen und ein besonders geeignetes Wachstumsmedium aus Molke bzw. Molkehydrolysat. In Schüttelkulturen sind so Mengen von ≥ 100 mg/l FXIIIa erzielbar, in Fermentation nach "scale-up" ≥ 500 mg/l.

Der Gerinnungsfaktor XIII (FXIII) bildet das Endglied der Gerinnungskaskade beim natürlichen Prozess der Blutgerinnung. FXIII wurde 1944 als Serumfaktor erstmals von Robbins beschrieben. Robbins hatte beobachtet, daß Fibringerinsel aus normalem Vollblut in Harnstofflösung unlöslich sind, während Koagula aus hochgereinigtem Fibrinogen und Thrombin löslich sind.

Das Molekulargewicht von FXIII aus Plasma beträgt ca. 300 kDal. Das Plasmaprotein besteht aus der Untereinheit a von ca. 80 kDal und der Untereinheit b von ca. 75 kDal und existiert als Tetramer mit je zwei a und b Untereinheiten (a2b2). Die Untereinheit a (FXIIIa), enthält die enzymatische Aktivität. FXIIIa wird durch Abspaltung eines 4 kDal großen aminoterminalen Peptides durch Thrombin zu FXIIIa' aktiviert. Diese aktivierte Form des FXIIIa fungiert in Gegenwart von Calciumionen als Transglutaminase und vernetzt über intermolekulare - (γ–Glutamyl-ε-Lysin-Bindungen Fibrinmonomere zu festeren Gerinseln.

Die cDNA sowie die erstmalige Expression von FXIIIa in E. coli, animalen Zellen und Hefen ist in EP 0 236 978 beschrieben. Die Ausbeuten an aktivem FXIIIa in Hefen sind allerdings in dem beschriebenen Verfahren mit 150 ng/ml recht niedrig.

FXIIIa kann in hohen Ausbeuten in E.coli exprimiert werden (Amann et al., Gene 69, 301-315 [1988]). Allerdings ist der größte Anteil des in E.coli synthetisierten FXIIIa unlöslich und damit enzymatisch nicht aktiv. Als Alternative zur heterologen Expression in Prokaryonten bietet sich die Synthese in eukaryotischen Zellen an. Die heterologe Expression von biologisch aktivem FXIIIa in CHO-Zellen ist bereits gezeigt worden (Zettlmeißl und Karges, (1989), XIIth Congress of the International Society on Thrombosis and Haemostasis, Tokyo). Doch liegen hier die Ausbeuten nicht so hoch, daß eine kostengünstige Produktion gewährleistet wäre.

Hefen eignen sich als Wirtszellen deshalb, weil sie einerseits genetisch gut für molekularbiologische Arbeiten charakterisiert sind, Klonierungs- und Expressionsvektoren vorhanden sind und sie als Eukaryonten über die typischen posttranslationalen Modifikationsmechanismen verfügen. Andererseits besitzen sie als Mikroorganismen eine recht kurze Generationszeit und es sind hinreichend Fermentationsverfahren entwikkelt worden, um Hefen in hoher Zelldichte anzuziehen. In der Europäischen Patentanmeldung EP A2 0 236 978 ist bereits beispielhaft gezeigt, daß man in Bäckerhefe biologisch aktiven FXIIIa herstellen kann. Auch in der Spalthefe Schizosaccharomycespombe kann mit Hilfe molekularbiologischer Techniken rekombinantes aktives FXIIIa synthetisiert werden (Bröker and Bäuml, FEBS Lett. 248, 105-110 [1989]). In den zwei beschriebenen Beispielen liegt die Ausbeute allerdings mit 0,15 bzw. 2 mg/l nicht in einem Bereich, der eine Herstellung wirtschaftlich erscheinen läßt. Höhere Ausbeuten sind in der EP-Anmeldung A2 0 268 772 beschrieben. Plasmide, bei denen die FXIIIa-Synthese unter der Kontrolle des starken glykolytischen TPI-Promotors steht, bewirken eine Syntheserate von 10 mg/l. Mit Hilfe des ADHII-Promotors werden 50 mg/l erreicht.

Es bestand daher die Aufgabe, die Expression von FXIIIa so zu verbessern, daß Werte von ≥ 100 mg/l erreicht werden. Mit solchen Expressionsraten ist die gentechnische Herstellung von FXIIIa kostengünstiger als die klassische Aufreinigung aus Plazenten.

Wir haben gefunden, daß der Einsatz eines hybriden Gal $_{UAS}$/Cycl Promotors, die Deletion der 5'-nichttranslatierten Region sowie einer Verkürzung der 3'-nichttranslatierten Region von FXIIIa cDNA im Expressionsvektor, Auswahl spezieller Hefestämme und Einsatz eines gut geeigneten Wachstumsmediums enthaltend Glukose und Galaktose einzeln oder in Kombination eine hohe Ausbeute an FXIIIa bewirken. Der hybride Gal $_{UAS}$/Cycl Promotor stammt aus pEMBLyex4 (Cesareni, G. und Murray, A.H. in Setlow, J.K. (ed.) Genetic Engineering, Vol. 9, 135-154, Plenum Publishing Corporation, 1987),

Repräsentative Vektoren enthalten die FXIII cDNA ohne den 5'-nichttranslatierten und den 3'-nichttranslatierten cDNA Bereich, den letzteren vorzugsweise gekürzt von 419 Basenpaare (bp) auf nur 120 bp. Die Synthese der speziellen Vektoren pMB307 und pMB330 ist in der Figur und in den Beispielen gezeigt. Der zugrundeliegende Basisvektorist vorstehend genannter Vektor pEMBLyex4, in dessen Polylinker o.g. verkürzte FXIIIa cDNA einligiert wurde.

pEMBLyex4 liegt episomal in Kopienzahl von etwa 50/Zelle in Hefe vor und trägt die Selektionsmarker Leu2d und Ura3, so daß Hefestämme mit dem Genotyp leu2 und ura3 komplementiert werden können;

durch Insertion des Trp1-Gens (siehe Beispiel 2) in pMB307 zu pMB307T werden auch trp1 Stämme komplementiert.

Für pMB307 waren die S.cerevisiae-Stämme AH22 und Cl3ABYS86 am besten geeignet, für pMB307T der Stamm 150-2B. AH22 ist in A.Hinnen et al., (Proc.Acad.Natl. Sci. USA (1978) 75, 1929-1933) beschrieben; Cl3ABYS83 wurde in EP-A1-0 327 797 offenbart und 150-2B von C.Baldari et al. (EMBO Journal, (1983), 6, 229-234L) beschrieben.

Durch Anwesenheit von Glukose im Medium wird die Induktion der Gal oder Gal/$_{UAS}$ Promotoren durch Galaktose reprimiert. Überraschenderweise steigt die Syntheserate sehr stark, wenn von Beginn der Fermentation an neben Glukose auch Galaktose im Medium vorliegt. Besonders gut ist hydrolysiertes Molkepulver geeignet, das zudem noch billig erhältlich ist im Vergleich zu reiner Galaktose. Eine Alternative ist Molkepulver unter Zusatz von β-Galaktosidase bzw. Laktose-spaltenden Mikroorganismen.

Die Erfindung betrifft folglich den Einsatz von hydrolysiertem Molkepulver in Verbindung mit Hefe-Expressionsplasmiden unter Kontrolle eines mit Galaktose-induzierbaren Promotors (1), vorzugsweise des hybriden Gal $_{UAS}$/Cycl-Promotors (2), bevorzugt wird ferner das pEMBL yex4-Vektorgerüst verwendet, in das die um die 5'nichttranslatierteRegion deletierte und im 3'-nichttranslatierten Bereich verkürzte cDNA von FXIIIa eingesetzt ist (3). Bevorzugte Stämme zur Expression sind die Stämme AH22 (4) und C13ABYS86 (5) für Leu- oder Ura-Selektion bzw. 150-2B für Trp-Selektion (6).

Besonders bevorzugt ist die Kombination vorstehend genannter Merkmale (1), (2), (3), (5) bzw. (6) zur Expressionsoptimierung von FXIIIa. Bei großtechnischer Durchführung in 100 1 oder größeren Fermentern wurden bei letztgenannten Verfahren FXIIIa-Konzentrationen ≥ 500 mg/l erreicht.

Die Erfindung ist ferner in den Beispielen und den Patentansprüchen beschrieben.


## Beispiele:

Die hier verwendeten molekularbiologischen Arbeitstechniken basieren vorwiegend auf Maniatis et al.: Molecular Cloning. A Laboratory Manual. Gold Spring Harbor Laboratory, Gold Spring Harbor (1982) und in bezug auf die Arbeiten mit Hefen vor allem auf Rodriguez et al.: Recombinant DNA Techniques. Addison-Wesley Publishing Company, London (1983). Als Wirte für die Synthese von FXIIIa wurden allgemein zugängliche Laborstämme der Bäckerhefe Saccharomyces cerevisiae verwendet, die ein oder mehrere Defekte in biosynthetischen Enzymen haben. Diese Defekte wurden durch die jeweils entsprechenden funktionstüchtigen Gene auf den Vektoren komplementiert. Die Transformation der Hefestämme erfolgte nach der LiCl-Methode, wie sie für die Spalthefe optimiert worden ist (Bröker, M., Biotechniques 5, 516 - 518 [1987]) mit der Modifikation, daß der Hitzeschock nicht bei 46°C, sondern bei 42°C durchgeführt wurde. Einzelne Klone können drei bis vier Tage nach der Transformation mit Plasmid-DNA auf YND-Minimalmedium selektioniert werden. Die Anzucht der Transformanten in Flüssigkultur zur Gewinnung von FXIIIa wurde wie folgt durchgeführt: 50 ml YNB-Medium in einem 250 ml-Erlenmeyerkolben mit seitlichen Schikanen wurden mit einer Einzelkolonie angeimpft und zwei Tage bei 30°C geschüttelt. Wenn ein Hefestamm benutzt wurde, dessen Gesamtheit der genetischen Defekte durch den Vektor nicht komplementiert wurde, wurde dem Medium 0,02 - 0,04 mg/l der entsprechenden Aminosäure oder der Base zugesetzt. 10 ml aus solch einer Vorkultur wurden zum Beimpfen von 100 ml komplexem YPD-Medium verwendet. Je nach verwendetem Plasmid und Hefestamm war der Plasmidverlust unter diesen Nichtselektionsbedingungen verschieden groß. Kulturen, bei denen die FXIIIa-Syntheseunter der Kontrolle des durch Galaktose regulierbaren Gal1 oder Gal $_{UAS}$/Cyc1-Hybridpromotor steht, wurden 24 Stunden nach dem Animpfen mit 2% Galaktose versetzt, um den Gal-Promotor zu induzieren.

Bäckerhefe kann auf Medium mit Galaktose als alleiniger Kohlenstoffquelle wachsen. Galaktose wird durch eine spezifische Galaktosepermease in die Zelle transportiert und wird letzlich als Glukose-1-phosphat in die Glykolyse eingeschleust. Hieran sind folgende Enzyme beteiligt: die Galaktokinase (Gal1), die -D-Galaktose-1-phosphat-uridyltransferase (Gal7) und die Uridin-Diphosphoglucose-4-epimerase (Gal10). Die Gene dieser Enzyme liegen dicht beisammen und werden koordiniert reguliert. Bei einem Wechsel von Glycerin oder Glukose zu Galaktose als C-Quelle wird die Transkription dieser Gene tausendfach verstärkt. In Abwesenheit von Galaktose werden Gal1, Gal7 und Gal10 durch das negative Regulatorprotein, das durch das Gal80-Gen kodiert ist, reprimiert. Für die Expression von Gal1, Gal7 und Gal10 wird ein positives Regulatorprotein benötigt, das durch das Gal4-Gen kodiert ist. Die Gal80 und Gal4 Proteine werden konstitutiv in geringen Mengen synthetisiert. Das Gal80 Protein bindet an Gal4 Protein und inhibiert dadurch eine Aktivierung der Gene durch das Gal4 Protein. Ein Induktor (Galaktose oder ein Derivat dieses Zuckers) bindet vermutlich an eine Stelle von Gal80, so daß die Affinität von Gal80 Protein zu Gal4 Protein reduziert wird und Gal4 frei wird. Gal4 bindet nun vermutlich mit seinem aminoterminalem Ende an den 5'-Bereichen von Gal1, Gal7 und Gal10 und bewirkt die verstärkte Transkription der Gene. Diese distalen DNA-Regulationselemente von Hefegenen werden 'upstream activating sequences' (UASs) genannt.

## Beispiel 1: Synthese das Plasmids pMB307

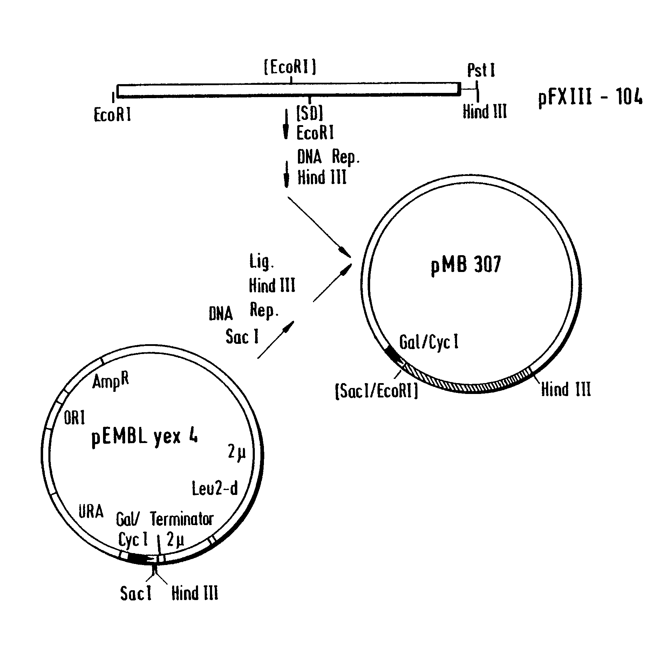

Startmaterial ist pFXIII-104 (Amann et al., Behring Inst. Mitt. (1988), 82, 35-42; Fig. 1) und pEMBLyex4; Das Plasmid pEMBLyex4 ist ein E.coli shuttle-Vektor. Wesentliche relevante Eigenschaften des Vektors sind, daß man ihn in E.coli aufgrund des Ampicillinresistenzgens selektionieren kann. In Hefen erfolgt die Selektion über das Ura3 oder Leu2-d Gen in Leucin und/oder Uracil auxotrophen Stämmen. Eine stabile Replikation in Hefen wird durch den Anteil der $2\mu$ DNA gewährleistet. Ein besonderes Kennzeichen des Vektors stellt der Gal-Cyc Hybridpromotor dar. 3'-endig dieses Elementes können Gene in den Polylinker insertiert werden, so daß in Hefen mit solch einem rekombinanten Plasmid die Expression des Fremdgens unter der Kontrolle des regulierbaren Gal-Cyc-Promotors steht. Eine effiziente Transkriptionstermination ist durch eine entsprechende DNA-Einheit gewährleistet, die sich 3'-endig vom Polylinker befindet. Der Zusammenbau von pMB307 ist in der Fig. schematisch dargestellt.

Ein Plasmid mit Deletion des 3'-nichttranslatierten Bereichs über 120 bp hinaus erzielte keine weitere Steigerung der Expression.

## Beispiel 2: Synthese des Plasmids pMB307T

Das Plasmid pMB307 trägt als Selektionsmarker die Gene Leu2-d und Ura3, womit Stämme, die den Genotyp leu2 und ura3 aufweisen, komplementiert werden. Eine Transformation mit einem modifizierten pMB307, das das Trp1-Gen trägt, weist den Vorteil auf, dar bei Stämmen mit dem Genotyp trp1 in komplexen Medien, die säurehydrolisierte Proteine als N-Quelle enthalten, ein sehr geringer Plasmidverlust eintritt. Die Aminosäure Tryptophan wird durch Säure leicht zerstört und ist in solchen Medien nicht mehr vorhanden, so daß selbst ein komplexes Medium hinsichtlich des Trp1-Markers ein Selektivmedium darstellt.

Der Vektor pMB307 wurde mit BstEll linearisiert und das 830 bp große Stul/EcoRI DNA-Fragment, das das Trp1-Gen trägt, aus dem Plasmid pGL2 (Das, S., Kellermann, E. and Hollenberg, C. J. Bacteriol. 158, 1165-1167 (1984) einligiert. Das neue Plasmid pMB307T kann nun auch trp1-Stämme komplementieren. Verschiedene Stämme wurden mit pMB307T transformiert und die FXIIIA-Ausbeute ermittelt. Die höchste Syntheserate von FXIIIa unter den getesteten Stämmen zeigte der Stamm 150-2B.

## Beispiel 4: Synthese des Plasmids pMB330

Die in EP 0 236 978 genannte cDNA von FXIIIa kodiert in Position 88 (CTC) für die Aminosäure Leucin. Ichinose et al. (1986), Biochemistry 25, 6900-6906) ermittelten in dieser Position hingegen ein TTC-Triplett, das für Phenylalanin kodiert. Die Aminosäuresequenz von FXIIIa, die von Takahashi et al. (1986), Proc. Natl. Acad. Sci. USA 83, 8019-8023 erstellt wurde, ergab an Position 88 ebenfalls ein Phenylalanin. Somit könnte es sein, daß die in EP 0 236 978 genannte abgeleitete Aminosäure Leucin in Position 88 ein relativ seltenes Allel im Vergleich zu Phenylalanin darstellt. Deshalb wurde ein FXIIIa-Expressionsvektor konstruiert (pMB330), der genauso aufgebaut ist ie das Plasmid pMB307 und sich nur darin unterscheidet, daß das CTC-Codon durch ein TTC-Codon ersetzt wurde, also in Position 88 für ein Phenylalanin kodiert.

## Beispiel 5: Einfluß verschiedener Stämme auf die Syntheserate von FXIIIa mit dem Plasmid pMB307 oder pMB307T

Das Plasmid pMB307 gewährleistet in S. cerevisiae 252 eine FXIIIa-Synthese unter den optimierten Kulturbedingungen von ca. 5 mg/l. Im Stamm HD war die Ausbeute an FXIIIa wesentlich geringer. Dies deutet darauf hin, daß eine hohe Syntheserate von FXIIIa nicht nur Plasmid- und Medienabhängig ist, sondern daß auch zelluläre Faktoren die Ausbeute mit beeinflussen können. Deshalb wurden mehrere Stämme individuell mit dem Vektor pMB307 transformiert und in Schüttelkulturen auf ihre Kapazität zur FXIIIa-Synthese hin analysiert. Das Ergebnis ist in Tab. 1 zusammengefaßt. Es zeigte sich, daß eine große Variabilität hinsichtlich der FXIIIa-Ausbeuten mit den einzelnen Stämmen erzielt werden können. Nur in Stämmen, die eine hohe Zelldichte erreichen und in denen der Plasmidverlust gering ist, konnte eine hohe FXIIIa-Synthese erzielt werden. Andererseits gewährleistet ein Stamm, der gut wächst und einen geringen Plasmidverlust aufweist, nicht zwangsläufig eine zufriedenstellende Ausbeute an FXIIIa. Erstaunlicherweise korreliert die Kapazität auch nicht mit wenig auxotrophen Markern; denn auch Stämme mit drei oder sogar fünf Defekten in biosynthetischen Enzymen erzielen beachtliche Ausbeuten von 5-7 mg FXIIIa/l. Unter den getesteten Stämmen fallen die Stämme AH22 und C13ABYS86 mit Ausbeuten von über 10 mg FXIIIa/l auf. Das Plasmid pMB307 trägt als Selektionsmarker die Gene Leu2-d und Ura3, womit Stämme, die den Genotyp leu2 und ura3 aufweisen, komplementiert werden. Eine Transformation mit einem modifizierten pMB307, das das Trp1-Gen trägt, weist den Vorteil auf, daß bei Stämmen mit dem Genotyp trp1 in komplexen Medien, die säurehydrolisierte Proteine als N-Quelle enthalten, ein sehr geringer Plasmidverlust eintritt. Die Aminosäure Tryptophan wird durch Säure leicht zerstört und ist in solchen Medien nicht mehr vorhanden, so daß selbst ein komplexes Medium hinsichtlich des Trp1-Markers ein Selektivmedium darstellt.

Der Vektor pMB307 wurde mit BstEll linearisiert und das 830 bp große Stul/EcoRI DNA-Fragment, das das Trp1-Gen trägt, aus dem Plasmid pGL2 (Das, S., Kellermann, E. and Hollenberg, C. J. Bacteriol. 158, 1165-1167 (1984) einligiert. Das neue Plasmid pMB307T kann nun auch trp1-Stämme komplementieren. Verschiedene Stämme wurden mit pMB307T transformiert und die FXIIIa-Ausbeute ermittelt. Unter den getesteten Stämmen zeigte der Stamm 150-2B mit 8 mg FXIIIa/l die höchste Syntheserate.

**Beispiel 6: Einfluß des Mediums nuf die FXIIIa-Ausbeute in pMB307-Transformanten**

Ausgehend von den Ausbeuten, die mit Hilfe des Plasmides pMB307 in Schüttelkulturen in üblichen Medien erzielt werden konnten, wurde versucht, durch Variation der Medienkomponenten die Syntheserate zu erhöhen. Die Transkription der FXIIIa spezifischen cDNA wird in Hefezellen, die das Plasmid pMB307 tragen, durch die Kohlenstoffquelle reguliert. In Medien, die nur Glukose enthalten, wird kein FXIIIa gebildet. Weiterhin wurde getestet, ob der Zeitpunkt der Induktion der FXIIIa spezifischen Transkription einen Einfluß auf die FXIIIa-Ausbeute hat, und ob die Energiequelle Glukose während der Wachstumsphase durch andere Kohlenstoffquellen ersetzt werden kann, ohne die Ausbeuten an FXIIIa zu verringern.

Ersetzt man die Glukose im YPD-Medium durch Maltose oder Glycerin, und füttert die Kulturen nach 24 h mit 2% Galaktose nach, so liegen auch hier die Ausbeuten bei ca. 2,5 bis 3 mg/l. Wenn hingegen Glukose im Medium von Beginn an durch Galaktose ersetzt ist, so liegt die Ausbeute an FXIIIa nur bei ca. 1 mg/l. Diese relativ geringe Ausbeute ist nicht auf vermindertes Wachstum zurückzuführen; denn die Zellzahl liegt mit ca. $5 \times 10^8$/ml genauso hoch wie in Kulturen, die nur Glukose enthielten oder nach 24 h mit Galaktose nachgefüttert wurden. Vermutlich wirkt sich eine Synthese von FXIIIa schon während der Wachstumsphase negativ auf den Stoffwechsel der Zellen aus und beeinträchtigt hierdurch die Synthese von FXIIIa während der Idiophase. Diese Beobachtung wird dadurch bestärkt, daß in Schüttelkulturen, in denen die FXIIIa-Synthese unter der Kontrolle des starken, aber konstitutiven ADC-Promotors steht, die Ausbeute an FXIIIa ebenfalls 1 mg/l nicht überstieg.

Die Ausbeute an FXIIIa in Hefen kann auf 4 mg/l erhöht werden, indem man nach 24 h, wenn keine Glukose mehr im Medium vorhanden ist, die FXIIIa-Synthese durch Zugabe von Galaktose nicht nur induziert, sondern nach weiteren 24 h und 48 h noch einmal Galaktose in einer Endkonzentration von jeweils 2% zusetzt. Wenn das komplexe Medium zu Beginn der Fermentation nicht nur Glukose enthält, sondern gleichzeitig zusätzlich Galaktose, und wenn täglich mit Galaktose gefüttert wird, so steigt die Produktionsrate sogar auf über 5 mg/l. Diese Versorgung durch Glukose bzw. Galaktose im YPD-Medium wird im folgenden als YPDG-Medium bezeichnet. Anscheinend wird die FXIIIa-Synthese durch Galaktose nicht induziert, so lange Glukose im Medium vorhanden ist. Andererseits kann die Umschaltung des Stoffwechsels nach Verbrauch der Glukose unter diesen Bedingungen sofort erfolgen und die Galaktose dient in der späten Wachstumsphase gleichzeitig als Energiequelle und als Induktor. Durch eine geschickte Komposition des Mediums konnte im Vergleich zu dem üblichen Standardmedium die Ausbeute an FXIIIa somit etwa verdoppelt werden.

**Beispiel 7: Molke und Molkehydrolysat als Galaktosequelle**

Die Synthese von FXIIIa ist durch die Vektoren pMB307 und pMB330 strikt durch Glukose im Medium reprimiert und wird durch Galaktose induziert. So lange Glukose im Medium vorhanden ist, wird offensichtlich auch in Gegenwart von Galaktose die Induktion verhindert. Da Galaktose ein teurer Zucker ist, wurde versucht, die Induktion nicht durch reine Galaktose zu vermitteln, sondern durch ein preiswertes Medium, das unter anderem auch Galaktose enthält. Hierzu wurde hydrolysiertes Molkenpulver der Fa. Molkerei-Zentrale Westfalen-Lippe eG, (Münster) getestet. Dieses Molkenpulver enthält außer Eiweiß, Fett und Mineralstoffen ca. 18% Glukose, 13% Galaktose und noch ca. 23% Laktose.

20 g hydrolysiertes Molkepulver wurden in 100 ml Wasser gerührt, auf 60°C erhitzt und somit in Lösung gebracht. Diese Präparation wurde 30 Minuten bei 10000 g zentrifugiert und anschließend der Überstand steril filtriert. Diese Lösung wurde entweder 1:1 mit sterilem Wasser verdünnt (A) oder 1:1 mit

5

zweifach konzentriertem YPD (B). Die FXIIIa-Ausbeute mit S.cerevisiae C13ABYS86 [pMB307 oder pMB330] betrug in Medium A nur ca. 5 mg/l. In Medium B wurden hingegen bis zu mehr als 100 mg FXIIIa/l nachgewiesen. Somit konnte gezeigt werden, daß Molkepulver, das in der Milchwirtschaft im Überschuß anfällt, und daher sehr preiswert ist, nach Hydrolyse der enthaltenen Laktose in Glukose und Galaktose als geeigneteSubstanz zur rekombinanten Synthese von FXIIIa eingesetzt werden kann. Bisher nicht näher identifizierte Substanzen bewirken sogar im Vergleich zu Kulturen, die mit reiner Galaktose induziert worden sind, eine erheblich höhere FXIIIa-Synthese. Die hohen FXIIIa-Ausbeuten konnten auch erreicht werden, wenn ein teilentmineralisierter Käsemolkensirup der Fa. De Melkindustrie Veghel, (BA Veghel, NL), eingesetzt wurde. Als weitere alternative Galaktosequelle bietet sich Molkepulver an, das zwar unbehandelt keine Galaktose enthält, aber Laktose. Diese, im Molkepulver befindliche Laktose, wurde nach Suspendieren des Pulvers in YPD mit β-Galaktosidase aus E.coli in Glukose und Galaktose hydrolysiert. Auch solch eine Präparation bewirkte eine hohe FxIIIa-Synthese in Hefen. Es war sogar möglich, die Hefekultur, die mit Molkenpulver supplementiert war, mit β-Galaktosidase aus E.coli oder Kluyveromyces lactis (in freier Form oder immobilisiert) zu versetzen, so daß erst während der Fermentation Galaktose durch Spaltung freigesetzt wurde.

Statt Molkehydrolysat als Galaktosequelle eignet sich auch Lactosehydrolysat für die Produktion von FXIIIa. Kommerziell erhältliche Lactosehydrolysate (z. B. Biolac GmbH, D-3221 Harbernsen oder IMA GmbH, D-6078 Zeppelinheim) bestehen zu ca. 50 % aus Gesamt-Zucker, wobei der Anteil an Glucose und Galaktose etwa 1 : 1 beträgt und neben den Monosacchariden auch noch ungespaltene Lactose in Konzentrationen von 5 - 15 % vorhanden ist. Solche Lactosehydrolysate können durch Autoklavieren sterilisiert werden, ohne daß sich Karamelisierungsprodukte bilden, die sich auf die Fermentation von rekombinanten Hefen negativ auswirken. Es wurde festgestellt, daß die Glucose, die in Molke- und Lactosehydrolysaten vorhanden ist, die Induktion der FXIIIa-Synthese und die Produktion von FXIIIa durch S. cerevisiae [pMB307] oder S. cerevisiae [pMB330] in keiner Weise die Induktion beeinträchtigt. Dieser Befund war unerwartet, da in der Literatur Glucose als Inhibitor für die Induktion des Galaktose-Operons beschrieben ist. Im weiteren kann auch sprühgetrocknetes Lactosehydrolysat anstelle von kommerziellen Lactosehydrolysatlösungen, nachdem es mit geeigneten Methoden sterilisiert worden ist, für die Fermentation eingesetzt werden. Da die hier beschriebenen hohen Ausbeuten an FXIIIa wurden sowohl mit Transformanten erzielt wurden, die entweder das Plasmid pMB307 oder pMB330 enthielten, kann man davon ausgehen, daß die hohen Syntheseraten nicht von bestimmten Allelen abhängig sind. Somit ist zu erwarten, daß auch bei anderen Austauschen von Aminosäuren als Leucin 88 gegen Phenylalanin 88 eine hohe Ausbeute mit diesem optimierten Produktionsverfahren gewährleistet ist.

Bei Umsetzung in 100-500 1 Fermentormaßstab mit o.g. Medium und sogenannten Plasmiden pMB307 oder pMB330 wurden Konzentrationen von FXIIIa ≥ 500 mg/l erreicht.

Der aufgereinigte FXIIIa war nach allen untersuchten Kriterien identisch mit FxIIIa aus Plazenten.

Tab. 1a

Ausbeuten an FXIIIa in verschiedenen Stämmen der Bäckerhefe

| Stamm | Defekte Gene | FXIIIa [mg/l] | Zellzahl [Millionen/ml] | Plasm.stab. [%] | $OD_{600}$ | pH |
|---|---|---|---|---|---|---|
| C13ABYS86 | leu2, ura3, his | 10 | 510 | 100 | 30.4 | 4.1 |
| Pep4-3 | leu2 | < 0.01 | 630 | 100 | 34 | 4.2 |
| Sc79 | leu2, trp1 | 1.5 | 1040 | 90 | 33 | 4.0 |
| Sc252 | leu2, ura3, ade1 | 5 | 380 | 100 | 25.4 | 4.8 |
| F809 | ura3, his4, ade6 | < 0.01 | n.d. | n.d. | 7.0 | 4.5 |
| 150-2B | leu2, ura3, trp1, his3 | 8 | 300 | 91 | 25.8 | 4.5 |
| AH22 | leu2, his4 | 10 | 460 | 86 | 34.5 | n.d. |
| 215 | leu2, his3 | 7.5 | 440 | 92 | 33.3 | 4.0 |
| 225 | ura3 | < 0.01 | 125 | 40 | 5.0 | 4.2 |
| 226 | ura3 | < 0.01 | 140 | 31 | 5.7 | 4.5 |
| 227 | ura3 | 0.1 | 140 | 58 | 15.7 | n.d. |

EP 0 431 543 A1

Tab. 1b

| | | | | | | |
|---|---|---|---|---|---|---|
| TD71.8 | leu2, ura3, his3, lys2, trp1 | < 0,01 | 240 | n.d. | 24 | 2,5 |
| 0A51 | leu2, trp1 | < 0,01 | 440 | 90 | 36,1 | 3,7 |
| D116.6 | leu2, trp1, his3 | < 0,01 | 320 | 60 | 26,2 | 3,8 |
| MGA3 | leu2, ura3, trp1 | 0,5 | 660 | 95 | 40 | 4,3 |
| 337 | ura3 | 0,4 | 300 | n.d. | 24,5 | 4,1 |
| F000 | leu2, ura3, his4, ade1 | 0,0 | 150 | n.d. | 26 | 4,5 |
| 00Y074 | leu2, ura3, his5, trp5 | < 0,01 | 570 | 30 | 33,5 | 4,5 |
| D744-9A | leu2, trp1, his3, lys1, arg4 | 5 | 635 | n.d. | 46,5 | 3,8 |
| U303-1A | leu2, ura3, trp1, his3, ade2 | 7 | 250 | 80 | 25,25 | 4,4 |
| TD103.1 | leu2, ura3, trp1, his3, ade1, arg4, lys2 | 6 | 250 | n.d. | 23 | 4,3 |
| KN79 | leu2, trp1 | < 0,01 | 960 | n.d. | 40 | 4,0 |
| 00Y913 | ura3, leu2, ade3, trp1, his3 | < 0,01 | 200 | n.d. | 37,25 | 4,5 |
| NKY290 | ura3, trp1 | 0,08 | 550 | n.d. | f | 4,6 |

n.d.: nicht durchgeführt; f: flokkuliert, nicht meßbar

**Legende zur Figur:**
**Konstruktion von pMB307**

Das FXIIIa-spezifische EcoRI/HindIII cDNA-Fragment wurde in den Polylinker von pEMBLyex4 ligiert. Im Vergleich zu der bereits beschriebenen FXIIIa cDNA (Amann et al., a.a.o.) sind die interne EcoRI-Stelle und die Shine-Dalgarno ähnliche Sequenz mutiert worden, ohne daß sich die Aminosäuresequenz ändert. Diese

EP 0 431 543 A1

Merkmale sind durch eckige Klammern dargestellt.

DNA Rep. bedeutet Auffüllen der überstehenden Enden, Lig. die Ligation des entstandenen Konstrukts.

pMB330 unterscheidet sich von pMB307 nur durch Phe88 (TTC) anstelle von Leu88 (CTC) innerhalb der FXIIIa-Sequenz.

## Ansprüche

1. Verfahren zur Expression von Fremdgenen in Hefen unter Verwendung eines durch Galaktose induzierbaren Promotors, dadurch gekennzeichnet, daß Laktosehydrolysat, hydrolysiertes Molkepulver eingesetzt oder Molkepulver unter Zusatz von $\beta$-Galaktosidase bzw. Laktose-spaltenden Mikroorganismen verwendet wird.

2. Verfahren nach Anspruch 1, dadurch gekennzeichnet, daß teilentmineralisierter Käsemolkesirup eingesetzt wird.

3. Verfahren nach Anspruch 1, dadurch gekennzeichnet, daß der Gal/Cycl-Hybridpromotor eingesetzt wird.

4. Verfahren nach Anspruch 1, dadurch gekennzeichnet, daß der Gal/Cycl-Hybridpromotor und die cDNA des um die 5'-nichttranslatierte Region deletierte und bei der 3'-nichttranslatierten Region von 419 auf 120 Basenpaaren reduzierte Faktor XIIIa eingesetzt wird.

5. Verfahren nach Anspruch 4, dadurch gekennzeichnet, daß ein Expressionsplasmid mit dem Selektionsmarkern leu2d, Ura3 und/oder Trp1 eingesetzt wird.

6. Verfahren nach Anspruch 5, dadurch gekennzeichnet, daß der Hefestamm AH22, C13ABYS86 oder 150-2B eingesetzt wird.

7. Verfahren zur Expression von Faktor XIIIa in Hefen mittels Promotoren von Hefe-Genen des katabolen Galaktosestoffwechsels.

8. Verfahren nach Anspruch 7, dadurch gekennzeichnet, daß Promotoren des Gal1 oder Gal10 Gens oder promotoraktive Teile derselben verwendet werden.

9

**Europäisches Patentamt**

# EUROPÄISCHER RECHERCHENBERICHT

Nummer der Anmeldung

## EP 90 12 3154

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,Y | EP-A-0 206 571   (THE UNIVERSITY OF KENTUCKY RESEARCH FOUNDATION) <br> * das ganze Dokument * <br> — — — | 1-2,3,5-6 | C 12 N <br> 15/54 <br> C 12 N 15/81 <br> C 12 N 1/18 |
| D,Y | EMBO JOURNAL. vol. 6, no. 1, 1987, EYNSHAM, OXFORD GB Seiten 229 - 234; BALDARI, C. et al.: "A novel leader peptide wich allows efficient secretion of a fragment of human interleukin 1 beta in Saccharomyces cerevisiae" <br> * das ganze Dokument * <br> — — — | 3,5-6 | C 12 N 1/21 // <br> (C 12 N 1/21; <br> C 12 R 1:865) |
| X | EP-A-0 012 501   (THE KROGER CO.) <br> * das ganze Dokument * <br> — — — | 1-2 | |
| D,X,A,D | EP-A-0 268 772   (ZYMOGENETICS INC.) <br> * Seite 7, Zeilen 2 - 26 * * Seite 4, Zeilen 24 - 40 * <br> — — — | 7-8,4 | |
| X | THE JOURNAL OF CELL BIOLOGY vol. 107, no.6, part 3, December 1988 <br> & HAAS, P.E.: "Expression of active human Factor XIII in Yeast <br> — — — | 7-8 | |
| | | | RECHERCHIERTE SACHGEBIETE (Int. Cl.5) |
| P,A | BIOTECHNOLOGY vol. 8, Juni 1990, NEW YORK US Seiten 543 - 546; RINAS, U. et al.: "Characterization of recombinant Factor XIIIa produced in Saccharomyces cerevisiae" <br> * Seite 545, "Experimental protocol" * <br> — — — — — | 1-8 | C 12 N |

Der vorliegende Recherchenbericht wurde für alle Patentansprüche erstellt

| Recherchenort | Abschlußdatum der Recherche | Prüfer |
|---|---|---|
| Den Haag | 13 März 91 | ANDRES S.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

&: Mitglied der gleichen Patentfamilie, übereinstimmendes Dokument